# EUROPEAN PATENT APPLICATION

(11) **EP 1 747 994 A1**
(43) Date of publication of application: **31.01.2007**
(21) Application number: 05291613.7
(22) Date of filing: 28.07.2005
(51) Int. Cl.: B65B 3/06

(54) **Serially linked containers for containing a sterile solution**

(71) Applicant: I.D.M. IMMUNO-DESIGNED MOLECULES, 75011 Paris (FR)
(72) Inventor: Johnson, Craig, 92619 Irvine CA (US)
(74) Representative: Grosset-Fournier, Chantal Catherine

(57) **Abstract**

The invention relates to a system which can be sterilized, for preparing individual containers to be filled with a substantially accurately predetermined volume of a sterile solution, which comprises at least one string of containers comprising at least two containers which are :
- serially linked or serially pre-attached to one another to form a single tubing,
- predefined to regulate individual container fill volume either by partial partition of said single tubing or by specification which is indicative of the boundaries of the individual containers, and
- made of a substantially non-deforming material,

one extremity of each of the at least one string of containers comprising a single inlet for the sterile solution and the other extremity comprising a single outlet for air and / or for the sterile solution,
said single tubing being the exclusive flowing means for the sterile solution and for air, when present, from the single inlet to the single outlet of the string of containers.

## Description

The present invention relates to serially linked containers for containing a sterile solution.

There are currently two types of systems for distributing aliquots of sterile pharmaceuticals :
- open systems which are implemented under tightly controlled environmental conditions, and
- closed systems which require minimal environmental controls.

Open systems are typically implemented in a biological cabinet or laminar flow cabinet which passes highly purified air in a defined direction across the work area. While this environment is the traditional use, it is susceptible to exposure to occasional viable particles, does not usually filter out many virus particles, and is expensive to maintain. Nevertheless, many open systems are currently in operation and use.

A first example of open system for distribution in controlled environments is the pipette, in the case of small volumes of bulk (less than about 20 ml). The pipette is not designed to work as a closed system. Using a pipette, fluid may reach the exit port and either plug the filter, be lost through the exit port or become non sterile. The working tip of the pipette is not connected in such a way that the incoming or exiting fluid is shielded from the environment in which it is being used. Furthermore, closure of the receptacle is usually manual. Thus, this system relies heavily on the sterile environment around it.

A second example of open system for distribution in controlled environments is the syringe, still in the case of small volumes of bulk (less than about 60 ml). The syringe or repeater pipette could be used in a nearly closed environment. However, the inside of the syringe barrel is exposed to the environment and the plunger maintains a resistive but not fully sealed closure during the draw of material into the barrel. Besides, the connection of the syringe or repeater pipette to the source and receptacle is typically done in an open manner. Moreover, closure of the receptacle may be done manually. As an open process, this system must be implemented in a tightly controlled environment in order to maintain sterile aliquots.

Automated systems are yet another example of open systems. These systems inject the material into an open vial which may then be closed using any of several options. Again, because of the open nature of the process, the fill must be completed in a tightly controlled environment. Such systems typically do not allow for single use sterile disposable sets. Thus cleaning and cleaning validation may add risk to concerns about cross contamination of products. Setup for these systems is also typically not practical for small numbers of aliquots. Maintenance and validation of the systems may also be relatively complicated for small volume distributions.

Regarding closed systems, these have also been commonly used in laboratories around the world. Usually, closed systems which are in use in laboratories connect each aliquot in parallel to the source. The connection may be made prior to sterilization or by sterile connection of pre-sterilized sets.

US patent No. 4,937,194, assigned to Baxter International, discloses a typical closed system for the metering of nutrient media into cell culture containers placed in parallel. US patent No. 4,021,283 discloses a method of making aseptic packaging, consisting of forming a web of bags, filling them with a sterile product and sealing them without exposing the sterile product to the ambient air. Yet, although this system of interconnected bags is closed by itself, it does not avoid the drawbacks of an open system since the containers have to be filled by introducing a filling pipe into the web of bags.

Canadian application No. 2,025,498 discloses another example of a bag set design, method and apparatus for preparing bags of sterile solution. This document provides for a long, tubular bag element, which can be filled with a sterile solution through a sterilized filter. A major inconvenience of this closed system is that particular care has to be taken if the precision of the volume of solution in each container is important: a weighing pan or a flow meter has then to be used to meter a defined volume or weight of the sterile solution.

International application No. WO 2005/030586 discloses a container filling assembly for sterilely filling containers within a closed system. This system includes a single inlet for supplying a fluid to the containers, connecting means that branch out to each individual container, and a vacuum source for driving the fluid inside the containers. The use of a vacuum source proposed by this document raises the problem of a possible leakage of ambient air into the vials, compromising the sterility of one or more vials, and a possible leakage of the fluid into the vacuum source. Besides, if the whole set of containers cannot be entirely filled, as is for instance the case when the reservoir of fluid is insufficiently filled with fluid, several containers (and not just one container) will then probably be only partially filled with fluid, which will not enable to obtain a set of accurately filled containers. This inconvenience is inherent in *parallel* container filling assemblies, such as the hub and spoke container assembly of WO 2005/030586, wherein all containers are to be filled essentially simultaneously. Additionally, fluid losses in the tubing set will be substantial for small volume fills and the sheer associated with filling under vacuum may be disadvantageous to sheer sensitive products such as cellular products.

Furthermore, a system for distributing aliquots of sterile pharmaceuticals may need to be frozen for storage. In this respect, the technical issue of providing a set of individual containers liable to be filled with a sterile solution and then to be frozen (down to the temperature of liquid nitrogen) and thawed, has not been correctly addressed by prior art methods either. One standard practice includes methods of injecting aliquots into open vials which are then closed with screw on caps. The requirement for manual manipulation makes this process unduly susceptible to errors such as spilling, sterility breeches, cross contamination, and lot mix-up prior to labeling. These systems are also susceptible to contamination during storage in liquid nitrogen by transmission through caps which may not be securely sealed. On occasion, such containers may leak, crack, or explode upon thawing as internal liquid phase nitrogen rapidly converts to the gas phase. A more closed system is also commonly used which incorporates closed bags. Such bags when filled commonly contain pockets of air which must be removed manually to prevent leaking, splitting and explosion upon thawing as described above. This system is also limited to volumes greater than about 10 mL since for smaller volumes, accuracy and losses become substantial.

In other terms, the technical issue of providing a system for rapidly filling a number of containers with an accurately predetermined volume of solution in sterile conditions, said containers being optionally well adapted for subsequent freezing and thawing, has not been satisfactorily addressed by prior art.

### Description of the invention

One object of the invention is to provide a system with a single use disposable set, for the sterile distribution of liquid material (notably pharmaceuticals or biological products in a sterile environment) into smaller aliquots, the volume of liquid material in each aliquot being substantially accurately determined by the controlled volume of receptacle containers without using any metering device.

Another object of the invention is to provide a set of containers filled from a single fill which may be stored in a connected state if desired.

Another object of the invention is to provide a method for rapidly filling many containers with fixed volumes in a substantially closed environment with minimal if any waste.

Another object of the invention is to provide a method of filling where air bubbles are substantially avoided and where filled containers are well adapted for subsequent freezing (potentially to cryogenic temperatures) and thawing.

Another object of the invention is to provide a method of filling which can be implemented for example in a laboratory but also on a commercial or even industrial scale, in the context of preparing relatively large numbers of aliquots of sterile solution.

The different objects of the invention are achieved by providing a chain of aliquot containers pre-connected in a serial fashion, the individual volume of which is predetermined by container design or by applied forces, and by filling the serially connected containers via a single flow of sterile solution, in particular opposite the direction of the gravitational or inertial forces.

The invention relates to a system which can be sterilized, for preparing individual containers to be filled with a substantially accurately predetermined volume of a sterile solution, which comprises at least one string of containers comprising at least two containers which are :
- serially linked or serially pre-attached to one another to form a single tubing,
- predefined to regulate individual container fill volume either by partial partition of said single tubing or by specification which is indicative of the boundaries of the individual containers, and
- made of a substantially non-deforming material,
   one extremity of each of the at least one string of containers comprising a single inlet for the sterile solution and the other extremity comprising a single outlet for air and / or for the sterile solution,
   said single tubing being the exclusive flowing means for the sterile solution and for air, when present, from the single inlet to the single outlet of the string of containers.

Examples of a sterile solution to be used according to the invention are cellular suspensions, diagnostic particle suspensions, and single access injectable pharmaceutical products.

By "string" is also meant a chain or series or sequence ; by "containers" is also meant aliquots or receptacles, especially those that are adapted for use in the context of the handling, storage and subsequent use of a sterile solution, especially in the medical or scientific context.

By "serially linked" is meant that the string of containers is in fact only one continuous piece of tubular material without internal connections, in which the individual containers are only virtually delimited but not physically delimited.

By "serially pre-attached" is meant that the string of containers is composed of physically distinct pieces of tubular material which are serially attached or connected *via* connections.

The serial nature of the linkage, respectively of the pre-attachment, refers to a configuration in which each container is linked, respectively pre-attached, to two neighboring containers on two respective sides, except the two containers that are at the respective extremities (inlet and outlet) of the string and that are in contact with only one other container.

By "single tubing" is meant a single stretch of material or materials forming a hollow vessel. The single tubing may be made either of several serially linked tubes or of several serially pre-attached tubes. If the single tubing is made of several serially pre-attached tubes, there may be a longitudinal overlap between some tubes, for instance if one extremity of a tube of smaller diameter is embedded in the extremity of another tube of larger diameter.

By "predefined to regulate individual container fill volume" is meant that the dimensions and thus the volume of each container of the string of containers, once filled, correspond to fixed and predetermined values, even though :
- the string forms a single tubing which may be made of only one piece of tube, the individual containers being not necessarily physically delimited on the tubing ; and
- the dimensions and / or volume of each individual container not filled with sterile solution, i.e. in particular when empty or partially or totally filled with air, may be undefined or not accurately defined.

By "partial partition" of the tubing is meant either a partial transversal sealing of the tubing or any other physical delimitation of individual chambers or containers within the tubing, such as successive transversal walls partially obstructing the tubing.

By "specification which is indicative of the boundaries of the individual containers" is in particular meant either one of the following :
- marks on the outside of the tubing or in the inside of the tubing (in case the tubing is made of an at least partly transparent material) ;
- a predefined mold or fixture applied to the tubing ;
- a predetermined location or dimension ;
- a set of connections between different tube portions serially connected to form the single tubing.

By "exclusive flowing means" is meant that the system is not provided with any other flowing means for air and / or the sterile solution than the serially linked containers. The single tubing is the only flowing path for air and / or the sterile solution. In particular, there is no need for a fill tube to be inserted inside the string of containers to fill them. It has to be noted that the flow path which defines the direction of the air flow or of the flow of sterile solution can be a straight line but also a curved or a zigzag line according to particular configurations.

The tubing or its components can be made for instance of rigid or flexible PVC (polyvinyl chloride), ABS (acrylonitrile, butadiene, and styrene), PETG (polyethylene terephtalate glycol), EVA (ethylene vinyl acetate), Teflon, polycarbonate, ABS (acrylonitrile-butadiene-styrene) or other plastics, glass, metal etc.

The system of the invention can be provided :
- empty ; or
- partially or totally full of air ; or
- partially filled with a sterile solution or in the course of a process of filling with a sterile solution ; or
- full of sterile solution (possibly with bubbles of air).

It should be understood that all references that are made to "air" in the present application usually relate to the ambient air or atmospheric air but can also represent any other gas or combination of gases if the system of the invention is placed in or put in contact with another gas or combination of gases instead of the ambient air.

It should also be understood that all references to a "sterile solution" could also represent any non-sterile solution that is sterilized once the system has been filled with said solution.

In fact, in certain applications which involve filled containers of sterile materials, the material prior to filling is not sterile. In such applications, it is sometimes required to work in an environment where the bio-burden is controlled but rigorous aseptic technique is not required. In such applications, it is possible to either sterilize just prior to filling or to fill first and sterilize afterwards. Sterilization prior to filling can be accomplished by filtering, heating, radiation, or other means. Following sterilization, careful aseptic techniques and tight environment control are required as described above for any open processing steps in order to maintain sterility. Advantages of the invention described herein for this situation have been discussed above. Sterilization following filling can be accomplished by heat, radiation, or other techniques. In such an instance, the filling is done in a clean environment for the open steps to minimize the bio-burden. Following the fill, sterilization is accomplished by exposing the filled containers to heat or steam, radiation, or other mechanisms. Thus, the invention described herein may apply to such fills which are open or partially open during fill but which are closed when sterilized, because of the advantages associated with the clean and potentially biocompatible environment provided.

The invention more particularly relates to the above-mentioned system, further comprising means for air displacement and connecting means, said means for air displacement being connected to the single outlet of each of the at least one string of containers via said connecting means.

The means for air displacement are in particular useful for chasing air, if present, from the system at the time said system is being filled with the sterile solution.

Examples of "connecting means" are pre-connection by various weld or bonding methods, sterile connection devices such as the SCD devices made by Terumo® or single use sterile connecting devices.

In a particular embodiment, said means for air displacement comprise at least one pre-sterilized venting bag or at least one sterile barrier filter.

By "venting bag" is meant a container designed to be filled with air chased from the system when filling the at least one string of containers with the sterile solution.

By "sterile barrier filter" is meant a filter which is typically available commercially and which allows the air chased from the system when filling the at least one string of containers with the sterile solution to pass from the system without either exposing the sterile solution to environmental air contaminants or exposing environmental air to the solution contents.

The above-mentioned system advantageously further comprises at least one reservoir for sterile solution, said at least one reservoir of sterile solution being optionally connected to the single inlet of each of the at least one string of containers.

By "optionally connected" is meant that a system with a reservoir for sterile solution connected to the at least one string of containers as well as a system with a separate reservoir for sterile solution to be subsequently connected to the at least one string of containers are both within the scope of the present invention.

The advantage of having the reservoir connected to the system is that the connection step is not required at the time of fill. However, the advantage of not having the reservoir pre-connected, is that the reservoir may be handled separate from the filling system as may be preferred in the case of culturing or storage of the bulk sterile solution prior to fill.

Maintaining sterility during connection of reservoirs or other components which are not pre-connected, may be accomplished using devices such as spikes or interconnecting Luers in an aseptic environment or by using closed connecting systems such as heat-welded sterile connecting devices.

The system may comprise more than one reservoir for sterile solution, in particular if the sterile solution is obtained by mixing the contents of two or more reservoirs.

The system may comprise a sterilizing filter or other means of fluid sterilization between the outlet of the reservoir and the inlet of the at least one string of containers for sterilizing the content of the reservoir during the fill step, in which case the sterile solution is in fact considered sterile only once it has been injected into the at least one string of containers.

Examples of reservoirs include bags, pouches, burettes (optionally provided with an air filter), bottles etc.

Advantageously, when the sterile solution is present inside the system and in particular when the reservoir for sterile solution is full, the system is closed relative to the ambient environment, so that there is no contact between the sterile solution and air, when present, inside the system on the one hand and the ambient environment outside the system on the other hand.

Such a closed system can be typically provided with a venting bag and reservoir such as defined above.

According to an advantageous embodiment, the above-mentioned system comprises a single string of containers, means for air displacement and a single reservoir for sterile solution, the means for air displacement being connected to the single outlet of the single string of containers via connecting means, and the single reservoir for sterile solution being optionally connected via additional connecting means to the single inlet of the single string of containers.

The serial nature of this system implies a number of advantages relative to currently available systems in terms of filling the individual containers of the system with the sterile solution.
- The filling process is quick. Little time is also required to eliminate air from the containers. Speed of fill is a particularly important factor when dealing with suspensions such as cell suspensions which settle over time or where chemical or biological degradation may occur.
- Losses and waste are potentially minimized in the connecting pathways.
- Serial connections make filling easy. In the case of serial connections, the operation is like a classical production line : the operator directs the sterile solution into a string of containers. This string of containers is then managed as a single container as it is filled.
- The system and method of the invention allow a reduction in cost of the overall filling process.

Furthermore, the accuracy of the filling is based on the volume of the containers and does not rely on any device for metering the volume or weight of fluid to be injected.

According to another advantageous embodiment, the above-mentioned system comprises at least two strings of containers, means for air displacement and a single reservoir for sterile solution, the at least two strings of containers being placed in parallel, the means for air displacement being connected to the outlets of all the strings of containers via connecting means, and the single reservoir for sterile solution being optionally connected to the inlets of all the strings of containers via additional connecting means.

Alternatively, when two or more strings of containers are present, using an analogy with components in an electric circuit, any combination of parallel and serial associations of strings is a possible configuration for the system of the invention.

The advantage of parallel strings includes the ability to have a large number of containers in a short distance. For manual manipulation, a large number of containers in a short distance may be advantageous since the operator can reach each of the available containers during the fill and closure of containers. Another advantage may be the control of container volume particularly when a mold or fixture is used.

According to a more particular embodiment, said system comprises N strings of containers (N ≥ 2), N individual means for air displacement, and a single reservoir for sterile solution, the N strings of containers being placed in parallel and each of the N individual means for air displacement being connected to the outlet of one single string of containers among the N strings of containers via connecting means, and the single reservoir for sterile solution being connected to the inlets of the N strings of containers via additional connecting means.

In this embodiment, multiple strings of serially linked containers are connected in a parallel manner so that the multiple strings of serially linked containers can be filled in a manner similar to that described for a single string.

The parallel strings of serially linked containers can be filled either simultaneously or at different times (for instance by temporarily obstructing some of the strings while filling the others).

More particularly the above-mentioned system can comprise N strings of containers (N ≥ 2), wherein said connecting means are branched connecting means which comprise N+1 extremities, one of said N+1 extremities being connected to the means for air displacement and each of the other N extremities being connected to the outlet of a single one of the N strings of containers, and wherein said additional connecting means are branched additional connecting means which comprise N+1 extremities, one of said N+1 extremities being optionally connected to the single reservoir for sterile solution and each of the other N extremities being connected to the inlet of a single one of the N strings of containers.

According to a preferred embodiment, the said substantially non-deforming material is a substantially rigid material.

Examples of such "substantially rigid material" are the following: ABS, polycarbonate, glass, or metal.

According to an alternative preferred embodiment, the substantially non-deforming material is a flexible material whose deformation is limited by applied forces such as framing means or an externally applied mold which regulates the container volume.

Examples of such "flexible material" are the following : flexible PVC, thin sheets of Teflon, EVA.

The use of applied limiting structures renders the flexible material "substantially rigid" by preventing or limiting its deformation. More precisely the flexible material can be allowed to expand up to a certain maximal volume, said maximal volume being set by the framing means or externally applied mold. The use of these externally applied limits is a major factor of volume accuracy when filling the individual containers with the sterile solution and sets the volume largely independent of filling pressure.

Examples of such framing means or externally applied mold are : externally applied frames or fixtures which limit deformation from the outside of the container, framing which is integral to the container walls which limit the deformation of the container such as limitations imposed by the elastic nature of the wall material itself or an integral mesh in the walls of the container which has limiting elastic stretch, externally applied molds or tooling which define and limit the shape of the filled container prior to the final partitioning of the containers.

According to an advantageous embodiment, said substantially non-deforming material is sufficiently non-deforming in order for each container to be filled with a predetermined volume of said sterile solution with a better accuracy than 20%, in particular with a better accuracy than 10%, and in particular with a better accuracy than 5%.

The accuracy that can be expected depends in particular on the volume, shape, nature of materials, and assembly requirements of the individual containers. Bigger volumes usually yield a greater relative accuracy. Minimizing the complexity of shapes and the number of connections may improve accuracy since fewer variations are then introduced during assembly of the string of containers. Tubular or spherical containers are more structurally sound than containers with large broad flat surfaces made of comparable materials. Thus, round containers tend to be less prone to dimensional variance. Furthermore, round containers tend to have less variance in volume for minor distortions than containers with large broad flat surfaces. Making containers of a rigid material may also favor accuracy due to the minimal deformation of rigid containers. Moreover, assembly of the strings of containers usually introduces variation. Thus, containers well adapted for minimal manipulation will tend to have more accurate volumes.

According to an advantageous embodiment, the system comprises at least one reservoir for sterile solution which is made of a flexible material.

The use of a flexible reservoir for sterile solution facilitates the obtaining of a completely closed system. Indeed the reservoir for sterile solution filled with sterile solution can be emptied by flowing the sterile solution into the at least one string of containers without any need for a contact with the ambient environment, owing to the ability of the reservoir to deform.

According to an advantageous embodiment, the system comprises means for air displacement which comprise at least one venting bag, said at least one venting bag being made of a flexible material.

For example the venting bag can be a flexible bag or pouch initially substantially empty or collapsed, which is liable to inflate upon filling of the system with the sterile solution as the air is displaced.

According to a particular embodiment, all containers have the same volume.

According to another particular embodiment of the above-mentioned system, at least some of the containers have different volumes.

According to a preferred embodiment of the above-mentioned system, the volume of each individual container independently ranges from about 0.1 mL to about 200 mL.

According to a preferred embodiment of the above-mentioned system, the number of individual containers per string of containers independently ranges from 2 to about 100.

According to a preferred embodiment of the above-mentioned system, the number of strings of containers ranges from 1 to about 20.

According to a preferred embodiment of the above-mentioned system, the containers are selected among the group composed of vials, formed containers, pouches, or bags.

By "formed container" is meant a container which is made of one or more pieces of a substantially rigid plastic, which are preformed and if necessary assembled together, prior to filling. Examples include thermoformed, or injection molded parts.

According to a particular embodiment of the above-mentioned system, at least one of the individual containers further comprises at least one individual port per container for extracting sterile solution and optionally at least one other individual port per container for letting in air when sterile solution is extracted.

Such an individual port for extracting sterile solution may be for instance used as an access port for a syringe.

According to a particular embodiment of the above-mentioned system, the at least one string of containers is made of a single tube of constant cross-section, the containers being predefined by a specification which is indicative of the boundaries of the individual containers, each container optionally comprising one or two individual ports such as capped ports and in particular such as capped female Luer ports.

Such a system minimizes assembly time, expense and error. It also simplifies the design which may improve accuracy.

According to a particular embodiment of the above-mentioned system, the at least one string of containers is made of a single tubing comprising portions of tube alternated with flow-through couplings and in particular male-female Luer connections, the containers being partly predefined by a specification which is indicative of the boundaries of the individual containers and by said flow-through couplings, each container optionally comprising individual ports in particular for attachment to a capped sterile vent.

Flow through couplings facilitates filling with no air in the filled containers or their access ports. This may be particularly important for containers stored at cryogenic temperatures.

According to a particular embodiment of the above-mentioned system, the at least one string of containers is made of a single tubing comprising alternated portions of tubing of respectively larger and smaller cross-section, the individual containers are substantially predefined by the alternation of said portions of tubing and each individual container optionally comprises an individual port such as a capped port.

The smaller cross section between containers provides a segment of tubing for sealing which minimizes the losses in the connections between containers.

According to a particular embodiment of the above-mentioned system, the at least one string of containers is made of a substantially rigid material and the individual containers are substantially predefined as strings of formed containers by partial partition of the single tubing and optionally comprise at least one individual port such as a capped port.

Such a configuration can be accomplished by molding or forming, such as thermoforming, the pieces of a string of containers which are then sealed together leaving a flow path for filling and for venting air.

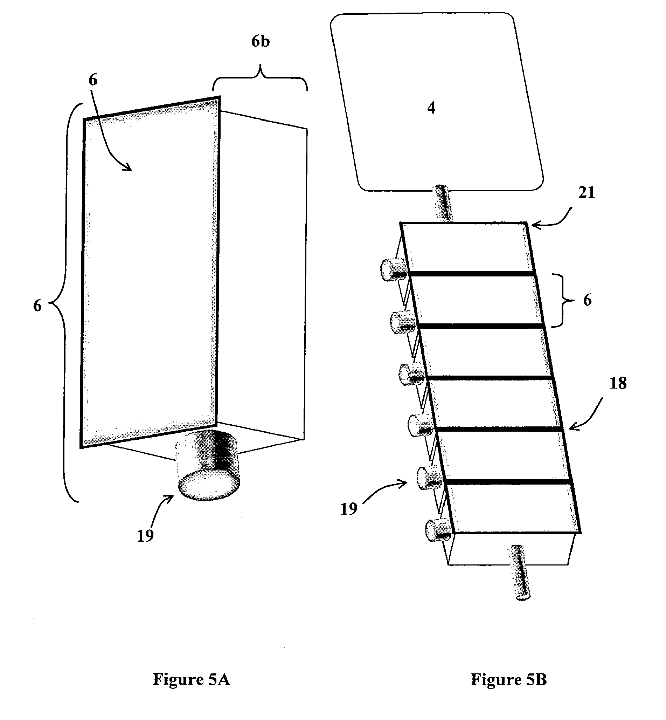

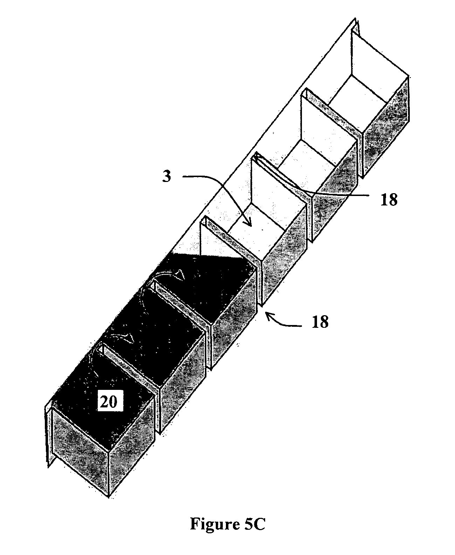

In a first instance, such a system could be composed of a formed strip which creates the primary container and flow path and a second piece which provides one side of the container and surfaces to seal the pieces together and to seal following the fill to partition the chambers. By sealing the edges of such an arrangement, a string of containers with flow path can be formed. Such a system is inexpensive to make and assemble (see Fig. 5).

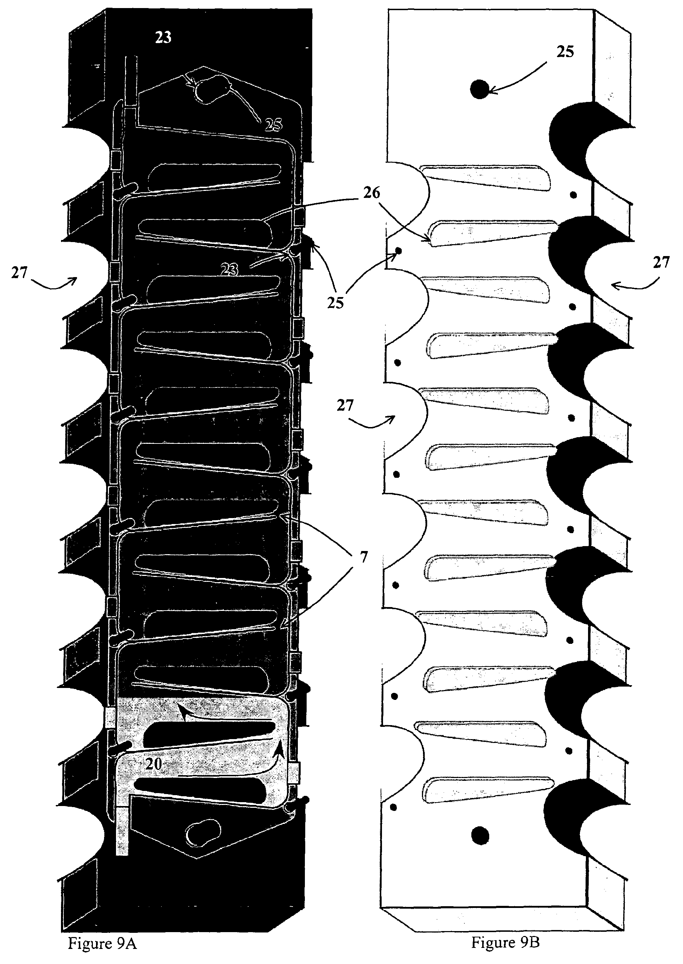

Another example of a string of formed containers can be created by designing longitudinal strips of the strings or containers which represent fractions of the string of containers. A set of molds or forms can be used to make strips which when sealed together, form complete strips of containers. A mold which forms half of the string of containers, divided longitudinally, forms a complete string of containers when two pieces are sealed together along their edges. Such a design can pre-form additional access ports into the system with minimal manual processing required. Such a design is inexpensive to make and assemble, simple to use, and very compact (see Fig. 9).

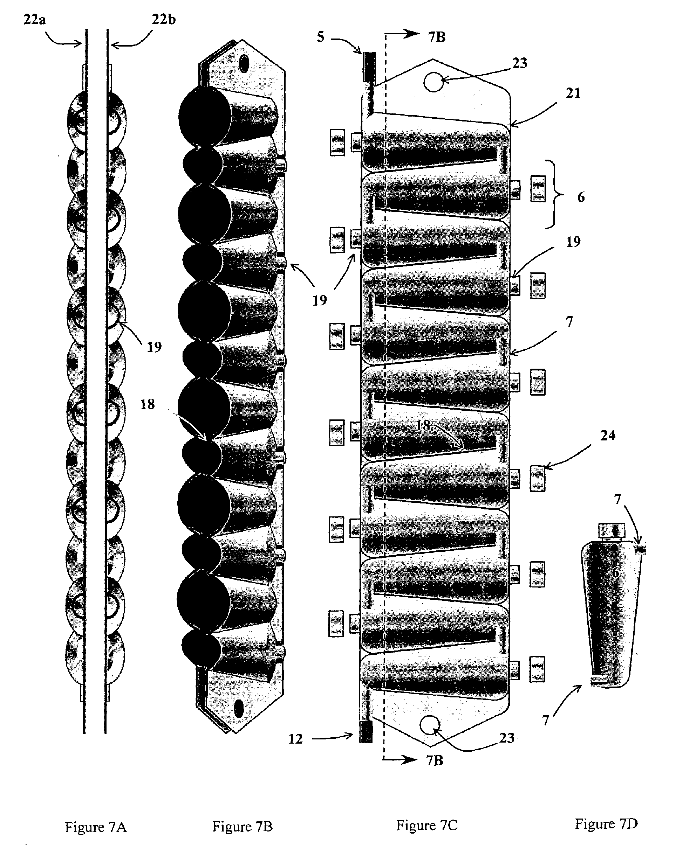

Another example of a strings of formed containers is a string of molded containers connected together end to end or side to side with a flow path from one container to the next. Such a system is also inexpensive to make, simple to assemble, and provides a flexible number of containers per string (see Fig. 7).

According to another particular embodiment of the above-mentioned system, the at least one string of containers is made of a flexible material which is rendered substantially non-deforming by applied forces such as framing means or an externally applied mold and the individual containers are predefined as flexible pouches by a specification which is indicative of the boundaries of the individual containers and / or by partial partition of the single tubing and optionally comprise at least one individual port such as a capped port.

According to this embodiment, the at least one string of containers is made of a flexible material formed as a single tube or pouch which is allowed to deform to the extent permitted by structures or fixtures such as framing means or molds in a manner which defines the volume of the individual containers which may be further defined by optional partial partitions of the single tubing and which optionally comprise at least one individual port such as a capped port.

According to a particular embodiment of the present invention, the containers are designed with materials and shapes which are resistant to cracking when cycled through freezing and thawing and which does not entrap air pockets within the containers their access ports or the fill ports.

Said freezing may involve a temperature of less than 0°C, and in particular a temperature below that of liquid nitrogen.

Said thawing typically involves a temperature such as room temperature (usually ~20°C) or body temperature (~37°C).

Examples of materials that are resistant to cracking include EVA, polyethylene, polypropylene, Teflon, and more. Device designs for applications frozen at very low temperatures should consider using similar thicknesses of contacting materials, similar coefficients of contraction of differing materials, materials with low glass transition temperatures, and more.

Typical shapes which optimize the containers' resistance to cracking are those which are well adapted to avoid the presence of air bubbles in the containers, i.e. simple shapes with as few corners, projections or cavities as possible. Containers with small distances from wall to wall in the cross section facilitate uniform freezing. Particularly, distance of ¼ inch (6.25mm) or less are preferred.

The invention also relates to a method for preparing individual containers to be filled with a substantially accurately predetermined volume of a sterile solution comprising the steps of :
- providing the above-mentioned system comprising at least one reservoir for sterile solution and additional connecting means, said at least one reservoir for sterile solution being connected to the single inlet of each of the at least one string of containers via said additional connecting means ;
- injecting the sterile solution from said at least one reservoir filled with sterile solution into the at least one string of containers via the single inlet of the at least one string of containers, so as to provide individual containers filled with a predetermined volume of sterile solution;
- sealing the at least one string of containers by sealing means at a plurality of positions, so as to provide filled, separate, individual containers ; and
- optionally detaching each individual container from the at least one string of containers by detaching means, so as to provide filled, separate, detached, individual containers.

By "sealing means" is for instance meant a dielectric welding tubing sealer such as one made by Sebra^{®}, or a laser, or a heat sealer.

By "detaching means" is meant means for breaking, cutting or tearing away connections to or between the sealed containers.

This method for preparing individual containers to be filled is advantageous relative to prior methods because it is quick, easy, accurate, it minimizes the connecting pathways and enables reduction in the costs of production.

According to a particular embodiment of the above-mentioned method, the system remains closed relative to the ambient environment during the steps of injecting the sterile solution into the at least one string of containers and of sealing the at least one string of containers, so that there is no contact between the sterile solution and air, when present, inside the system on the one hand and the ambient environment outside the system on the other hand.

According to an alternative embodiment, the system remains open relative to the ambient environment until the sealing of the at least one string of containers, the sterility of the sterile solution being maintained at all steps via at least one filtering means.

In this embodiment the reservoir for sterile solution or the means for air displacement or both are open or partially open to the ambient environment and provided with a filtering means such as a sterile barrier filter.

According to a particular embodiment of the above-mentioned method, the system comprises at least one venting bag for displaced air which is connected to the outlets of the at least one string of containers and which is mostly empty prior to injecting the sterile solution into the at least one string of containers.

The venting bag is then able to inflate and receive the air that is chased upon filling with the sterile solution.

According to a particular embodiment of the above-mentioned method, the injection of the sterile solution into the at least one string of containers is made from the top to the bottom or from the bottom to the top by means of hydrostatic pressure or of filling means such as a peristaltic pump.

Filling by hydrostatic pressure is either achieved by gravity of by the application of centripetal forces. Gravity can push the sterile solution from the bottom of the at least one string of containers to its top provided that the reservoir for sterile solution is placed above the inlet to said at least one string of containers.

Alternative filling means also include the application of a vacuum at the single outlet of the string of containers or at one of the possible additional ports.

According to a particular embodiment of the above-mentioned method, the injection of the sterile solution is made from the bottom to the top, whereby air bubbles in the containers are substantially avoided.

Avoiding air trapping is advantageous because trapped air may interfere with the volume accuracy and favor bursting of the container upon freezing at a temperature below that of nitrogen liquefaction and then thawing. According to the present invention, the air is displaced with the fluid, so that manual manipulation to express bubbles from the containers is generally not needed.

According to a particular embodiment of the above-mentioned method, the sealing of the containers of each of the at least one string of containers is carried out by sealing means either after the filling of said string of containers, or after the complete filling of all of the at least one string of containers.

The above-mentioned method advantageously comprises the additional final step of freezing and optionally thawing the sterile solution inside the containers, optionally more than once, without stress fractures appearing on said containers.

By "freezing" is meant placing at a temperature below 0°C or at a temperature as low as that of liquid nitrogen.

By "thawing" is in particular meant bringing to room temperature (usually ~ 20°C) or to body temperature (~37°C)

By "stress fractures" is meant cracking or breaking due to the stresses such as those associated with freezing, thawing, and normal handling while frozen.

The system described herein is able to provide accurate volume control without volume measurement, minimal losses for smaller volumes, rapid fills, and reduced risk of air entrapment with minimal manual manipulation. The containers are pre-connected to each other to prevent mix-ups prior to fill and labeling and the materials used may be designed to resist cracking when frozen and thawed.

Materials that might be used in this application include EVA, polyethylene, polypropylene, Teflon, and more. Device designs for applications frozen at very low temperatures should consider using similar thicknesses of contacting materials, similar coefficients of contraction of differing materials, materials with low glass transition temperatures, and more.

According to a particular embodiment of the above-mentioned method, the individual containers filled with a predetermined volume of sterile solution obtained after the step of injecting the sterile solution or the filled, separate, individual containers obtained after the step of sealing or the filled, separate, detached, individual containers obtained after the optional step of detaching are subsequently used for cellular or clinical applications.

Such cellular or clinical applications may include cellular vaccines, genetically altered cellular treatments, or viral gene therapy products.

### Description of the Figures

Figures 1 and 2 show two examples of small volume closed fill freezing straws (closed system dispensing) according to the invention. On the right (figures 1B and 2B) : a single string of containers with a venting bag for air displacement attached at the single outlet of the string. On the left (figures 1A and 2A) : an individual sealed and detached container.
Figure 3 shows an example of small volume closed fill freezing vials according to the invention. Figure 3B : a single string of containers with a venting bag attached for air displacement (at the outlet of the string) and a source bag or reservoir for sterile solution attached at the other end (i.e. at the inlet of the string). Figure 3A : an individual sealed and detached vial.
Figure 4 shows an example of a vial set assembly with several strings of containers placed in parallel, for a closed system dispensing according to the invention.
Figures 5A and 5B show an example of the string of formed containers configuration according to the invention (figure 5B: string of containers with venting bag attached; figure 5A: individual sealed and detached container). Figure 5C shows the filling process of the string of containers in the string of formed containers configuration.
Figure 6 shows an example of a set of flexible pouches according to the invention (figure 6B : string of containers with means for air displacement attached ; figure 6A : individual sealed and detached container).
Figure 7 shows another example of the string of formed containers configuration wherein the string is formed by two formed pieces. Figure 7A : top view (separated) ; Figure 7B : isometric cross-section of paired pieces (separated) ; Figure 7C : side view of paired pieces ; Figure 7D : individual container.
Figure 8 shows another example of set of flexible pouches according to the invention. The configuration is created by joining two sheets of flexible plastic material together. Figure 8A : top view ; Figure 8B : view in perspective ; Figure 8C : side view ; Figure 8D : individual container.
Figure 9A shows an example of set from Figure 8 positioned on a platen used for defining the volume capacity of each container. Figure 9B shows the opposite platen.

### Examples

### Serially connected vials

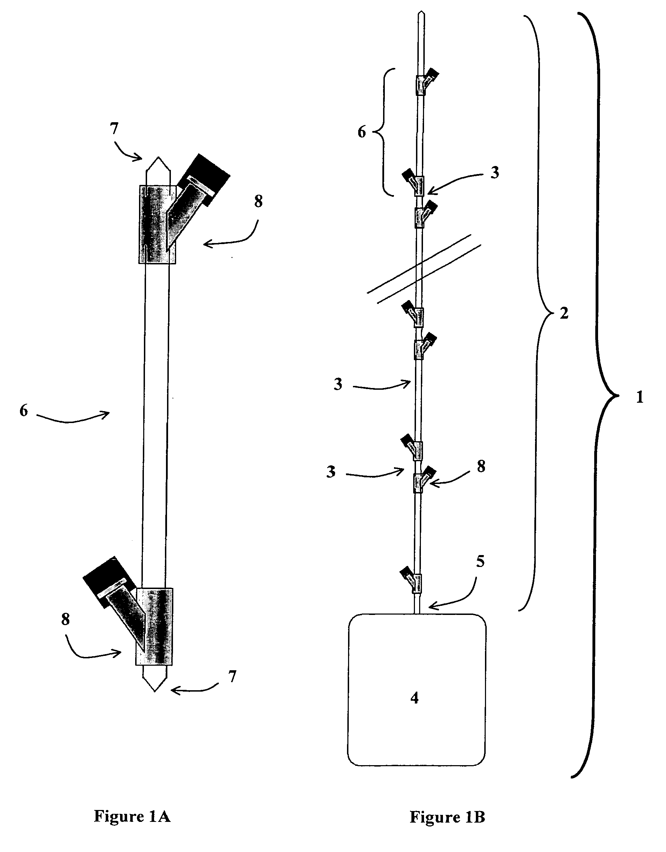

In the first instance of the system (1) according to the invention shown in figure 1 A and 1B, a single string (2) of serially connected vials is provided. This string is made of assembled pieces of tubing and connectors to form a single tubing (3) and is connected at its outlet to means for air displacement in the form of a venting bag (4) via connecting means (5). After filling each individual vial (6) can be delimited by two seals (7) performed transversally on the tubing (3) at the specified sites. Each individual vial (6) is provided with two Y connectors (8) that are initially closed. After filling and sealing, one of the Y connectors (8) can be opened (uncapped) to extract the sterile solution from the vial (6), for instance via a syringe. The other Y connector (8) can also be opened so as to provide air displacement during extraction via the syringe. Each Y connector (8) also provides a bubble trap to ensure that the sterile solution is not up to the rim of the vial (6) when the cap is removed.

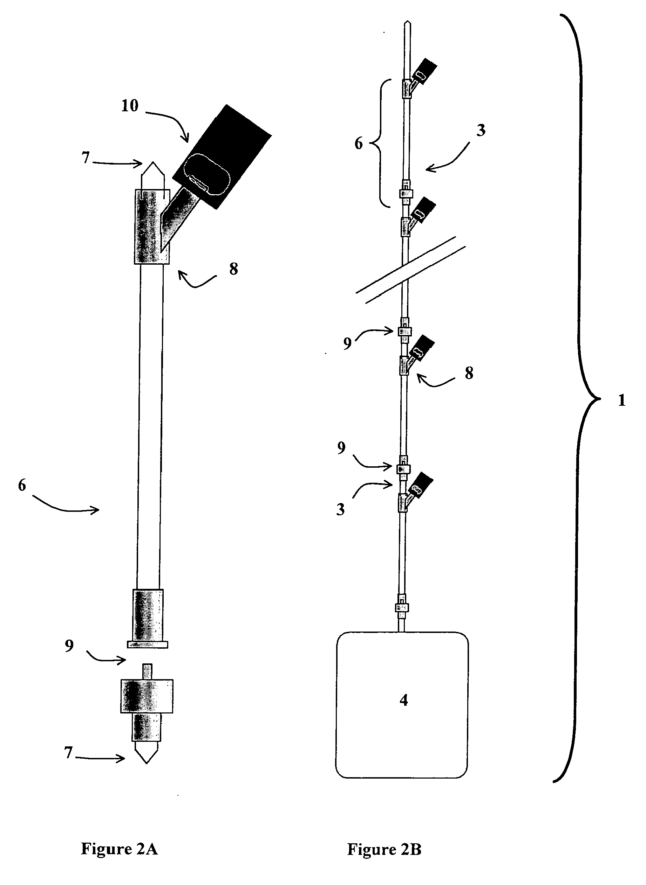

On figure 2A and 2B is shown another system (1), where individual containers (6) are pre-connected via male to female Luer connections (9). Each individual container (6) is also provided with a single Y connector (8) itself provided with a hydrophobic capped sterile vent (10). Individual containers (6) are thus delimited on the one hand by one of the Luer connections (9) and Y connectors (8) and on the other hand by seals (7) performed transversally on the tubing (3) after filling at the specified sites. A venting bag (4) is connected to the string of individual containers via another Luer connection.

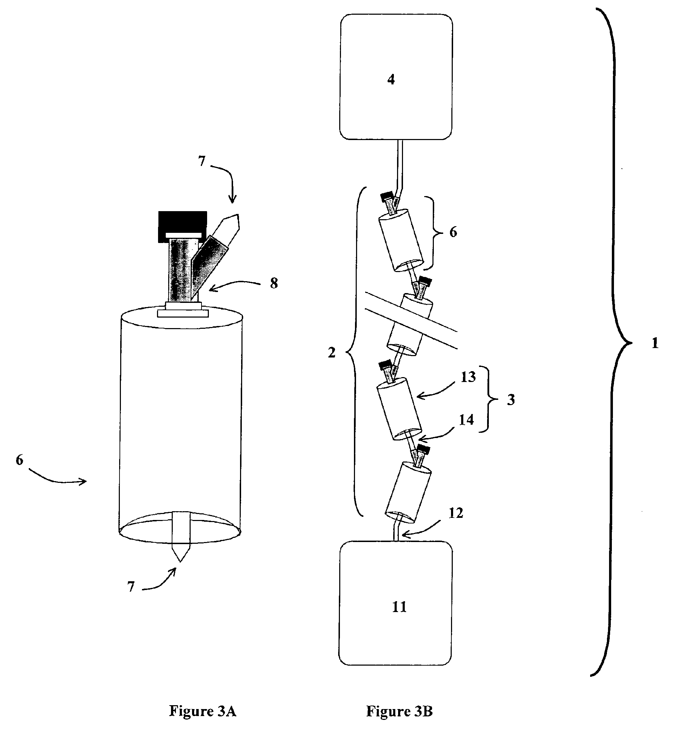

On figure 3A and 3B is shown yet another system (1) which is additionally provided with a reservoir for sterile solution (11) (or source bag) which is connected to the single inlet of the string (2) of containers (6) via additional connecting means (12), while a venting bag (4) is connected to the single outlet of said string. The part (13) of the tubing (3) which makes up the containers (6) is larger than the part (14) of the tubing (3) which makes up the serial connections between containers (6). Like in figure 2, individual containers (6) are delimited on the one hand by a Y connector (8) (Luer connection) and on the other hand by a seal (7) performed transversally on the tubing (3) after filling.

### Set of parallel strings of serially linked containers

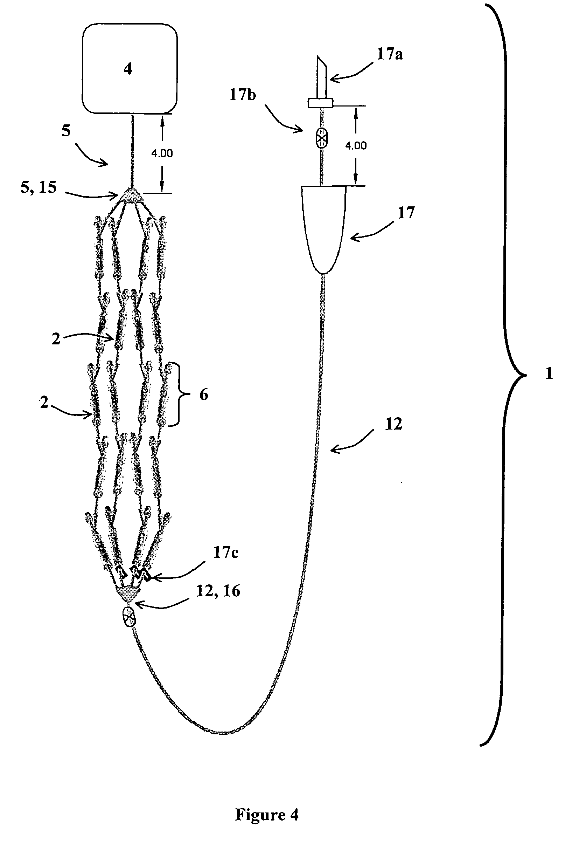

Figure 4 shows a system (1) according to the invention which is provided with a set of four strings (2) of containers (6) such as the one of figure 3 that are placed in parallel. These four strings are connected at their outlet to a venting bag (4) via connecting means (5) which comprise a common node with a 1 to 4 connector (15). They are also connected at their inlet to a reservoir for sterile solution (not shown) via additional connecting means (12) which typically comprise 30 inches sterile weld compatible tubing as well as a common node with an additional 1 to 4 connector (16). The system is also provided with a drip chamber (17) (or squeezable bubble trap) which helps prevent air bubbles from forming in the line when the containers (6) are filled and which works in conjunction with a clamp or check valve (17b) to allow pumping when sufficient head pressure is not available to clear the fluid from the line into the containers. The additional connecting means to the reservoir include a spike (17a) which is provided for connections when sterile welding is not possible or desired. Various tubing clamps (17c) are also provided to facilitate flow control into the string of containers which is not blocked.

### String of formed containers configuration

In the example of figures 5A and 5B, the string of individual containers (6) is a string of formed containers with 2 parts : a primary portion which forms multiple containers in a single stretch (6b) and a cover piece (6a) which seals each container. A seal is applied around the edge of the string of formed containers (21) and the boundaries (18) between the containers (6) are either partially sealed or not sealed at all to the cover piece. After filling, the connecting boundaries (18) are completely sealed. Each container (6) is provided with an access port (19). These ports may be protected by a separate punch-through membrane or the container material itself. This gives a sterile point for subsequently accessing the content of each formed container (6). It is of note that even though there may be partial seals (partial walls) between individual containers at the location of connecting boundaries (18), the serially linked containers are still made of a single tubing (3) which is the exclusive flowing means for air and for the sterile solution (20), as is shown on figure 5C.

Outer edges are pre-sealed. Partial partitioning seals which further define the container may be completed prior to fill. Only a small opening may be needed to pass fluid and gases into the linked containers. The set is typically sterilized prior to dispensing. During dispensing, the assembly is oriented with an incline sufficient to fill lower units first (the direction of fill is indicated by arrows on figure 5C). Air is vented out the top into the bag (4). Alternatively, in case of a more open system, air could be vented out through a sterile barrier filter. Seals between chambers are sealed after filling. The shape of the chambers may be adjusted to limit thickness if cryopreservation is required.

### Flexible pouches configuration

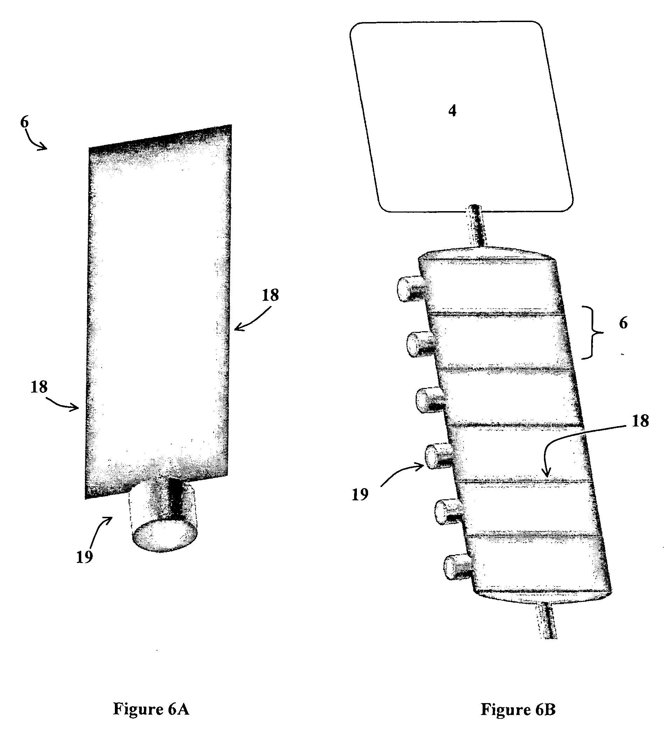

On figure 6A and 6B the individual containers (6) are flexible pouches that are connected so that the outer edges of the material are sealed and either part or none of the connecting boundaries (18) are sealed prior to filling. During the fill, the volume may be controlled by supplying sufficient pressure or by external form fitting using vacuum if necessary. Once filled, the boundaries (18) are sealed.

### String of formed containers configuration with 2 formed parts

Figures 7A, 7B, 7C, and 7D illustrate a string of serially linked containers created from 2 formed pieces (22a and 22b). The two pieces shown could be thermoformed and cut from 2 sheets of semi-rigid plastic.

After forming and cutting, pairs of parts are bonded together along the edges (21) and along most of each boundary between individual containers (18) while leaving the flow through path unsealed. Alignment holes (23) are provided in order to facilitate the bonding and to orient the string of containers during filling. The assembly forms cavities as illustrated in the expanded isometric cross section view (Figure 7B). The bonded surfaces (boundaries) are not emphasized but are represented by the dark lines between the containers (18) on the side view (Figure 7C). These bonded regions can also be perforated to facilitate separation of the parts after filling. The ports (19) can be reinforced with a collar to hold them in place. The collar can be designed to accommodate a closure (24) such as a threaded cap, an access septum for needle access or needle free access, a tubing spike, a slip connect cap, or tubing. The closure (24) is normally added prior to sterilization. On Figure 7C are also shown the additional connecting means (12) which connect the string of containers to a reservoir of sterile solution (not shown) and connecting means (5) to a venting bag or filter (not shown). The two seals (7) applied across the flow through path on a filled individual container (6) are shown in Figure 7D.

Note that the containers are linked with pass through features which allow fluid to flow into the lower container and through the high-point in the lower container into the adjacent container above it. Having the ports oriented horizontally or nearly horizontally (as illustrated), also minimizes the air trapped in the ports.

During filling, the system can be positioned in a filling fixture to hold the position but may allow manual access to manipulate if needed. The fluid is added from the additional connecting means (12). Each container is filled sequentially by filling and then overflowing into the adjacent container as air is pushed out of the port shown at the top of the drawing (connecting means 5). The device may need to be rocked during filling to ensure complete air removal. Following filling, the pass through ports are sealed (7) and the vials are ready to separate into individual containers (6). The ease of removal of air is important for applications where freezing to temperatures near liquid nitrogen is required.

### Particular flexible pouch configuration and fixture

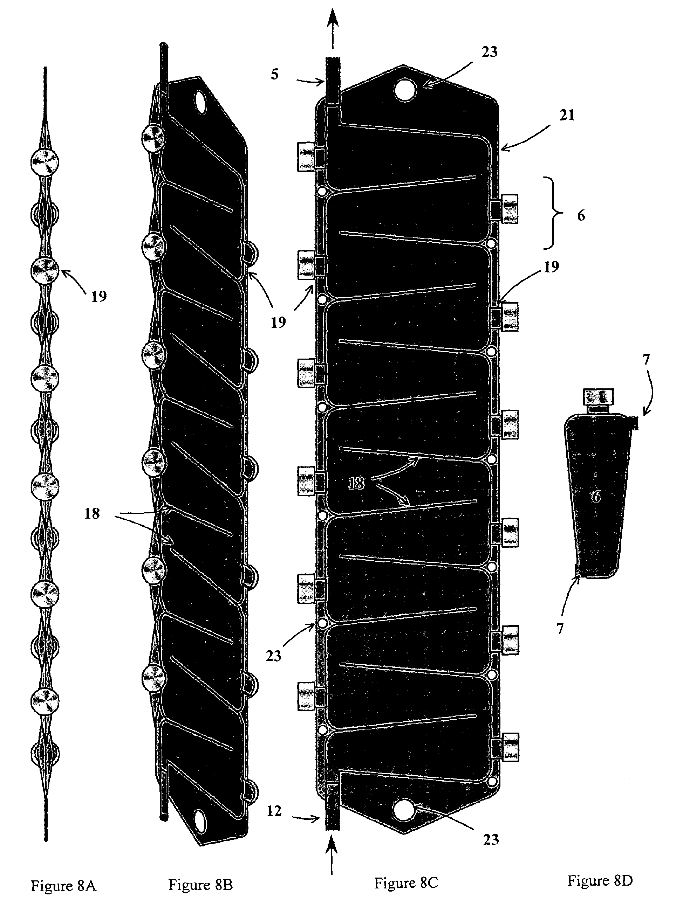

Figures 8A, 8B, 8C, and 8D illustrate a flexible pouch system composed of a single string of serially-linked containers (6). This configuration is created by laying two sheets of flexible plastic material together and applying a tool that seals along the edges (dark lines) (21) and along the boundaries between the individual containers (18), perforating along the sealed boundaries between the individual containers (18) to facilitate later separation, and sealing and cutting the outline form of the bag along the edges (21) from the sheets. This can be accomplished in a single step.

Container access ports (19) are then inserted along the edge of the bag as shown and bonded or welded in place. These ports can be closed with threaded caps, septum's for needle or needle free access, slip-lock closures, spike ports, or tubing. The fill tubing (additional connecting means 12) is also bonded in place at one extremity and a venting tubing (connecting means 5) at the other extremity. The arrows indicate the direction for filling and venting. On Figure 8D is shown a filled and detached individual container (6), with the flow through ports sealed (7).

Alignment holes (23) are provided which allow the bag to be positioned to a tool such as that shown in Figure 9A and 9B to improve the accuracy during filling and to control the final fill volume.

When it is desired to fill the container to its full capacity, container volume can be limited by the elastic nature of the materials used which are limiting at low pressures. To fill the container partially or when the container material is more elastic, the container volume can be limited by applying a fixture that controls the available space for filling.

Figure 9A shows the set from Figures 8A, 8B, 8C and 8D positioned on a platen which could be used to define the volume capacity of the bag. The opposing platen is shown in Figure 9B. On the surface of these platens are guides (25) that align with the alignment holes (23) in the part to hold the bags in position and to line up the opposing platen. Optional embossed shapes (26) across the surface of one platen line up with the mirror image embossment on the opposing platen. When the platens are pressed against each other with the bag in between them, the embossment modifies the capacity of the containers. It is also possible to control the container volume by using spacers between the platens with or without the embossment. Access groves (27) are provided to allow manual manipulation, clearance for ports or tubing, and for access to seal the flow through ports between the containers (7). If at least one of the platens is transparent, the progress of the fluid and air can be easily monitored.

By orienting the bag with the vent end up and the input end down, the fluid (20) fills the containers sequentially starting from the bottom (see arrows). The fluid exits the lower container at the high point and begins to fill the adjacent container. As the device fills, the air is forced out of the venting port. Areas that trap bubbles are minimal. However, rocking may be helpful to ensure good air removal.

The tool may be applied prior to filling to restrict the volume during the fill or it may be applied following the fill to express excess fluid back out through the inlet port.

Once the containers are filled, the flow paths between the containers are sealed (7), the system is removed from its fixture, and containers are ready to be separated along the container boundaries (18).

There are several distinct advantages of this design.
o For a single bag design, containers of different volumes can be created by changing the spacing or embossment of the tool. This is particularly important in small volume production settings to minimize device production costs.
o The design and the tool can be easily adapted for thicker materials which are more compatible with low temperature freezing or thinner materials which are desirable when gas exchange is important.
o Device production is inexpensive and repeatable even for small volumes.
o In this design there is almost no waste of fill liquid in connecting tubing.
o Filling is simple and quick.
o Air removal is accomplished rapidly and reliably. The ease of removal of air is important for applications where freezing to temperatures near liquid nitrogen is required.
o It is possible to have a thin cross section without giving up accuracy. Thin cross sections also facilitates freezing in relevant applications.
o Containers have additional capacity beyond that which is filled during the fill. This is important for applications where the contents must be diluted or mixed with other fluids prior to extraction for use.

### Demonstration of filling a prototype and using it in a cryopreserved state

Prototypes have been built according to the model of figure 3B. Vials were made of PVC tubing and polycarbonate Y connectors.

The vials were filled from below and air was trapped in the capped arm as expected but the vials could be easily filled from the bottom up. Volumes were predictable based on internal diameter and length of the tubing. By filling the vials to the top and then starting to sealing from the top, less waste was observed.

To test the potential for use in a cryopreserved state, the filled vials were sealed off and frozen in the -80°C freezer over night. The following day, the vials were removed and evaluated for stress fractures. None were observed.

## Claims

1. A system which can be sterilized, for preparing individual containers to be filled with a substantially accurately predetermined volume of a sterile solution, which comprises at least one string of containers comprising at least two containers which are :
- serially linked or serially pre-attached to one another to form a single tubing,
- predefined to regulate individual container fill volume either by partial partition of said single tubing or by specification which is indicative of the boundaries of the individual containers, and
- made of a substantially non-deforming material,
one extremity of each of the at least one string of containers comprising a single inlet for the sterile solution and the other extremity comprising a single outlet for air and / or for the sterile solution,
said single tubing being the exclusive flowing means for the sterile solution and for air, when present, from the single inlet to the single outlet of the string of containers.

2. The system of claim 1, further comprising means for air displacement and connecting means, said means for air displacement being connected to the single outlet of each of the at least one string of containers via said connecting means.

3. The system of claim 2, wherein said means for air displacement comprise at least one pre-sterilized venting bag or at least one sterile barrier filter.

4. The system of one of claims 1 to 3, further comprising at least one reservoir for sterile solution, said at least one reservoir of sterile solution being optionally connected to the single inlet of each of the at least one string of containers.

5. The system of claim 1 to 4, **characterized in that**, when the sterile solution is present inside the system and in particular when the reservoir for sterile solution is full, said system is closed relative to the ambient environment, so that there is no contact between the sterile solution and air, when present, inside the system on the one hand and the ambient environment outside the system on the other hand.

6. The system of one of claims 1 to 5, comprising a single string of containers, means for air displacement and a single reservoir for sterile solution, the means for air displacement being connected to the single outlet of the single string of containers via connecting means, and the single reservoir for sterile solution being optionally connected via additional connecting means to the single inlet of the single string of containers.

7. The system of one of claims 1 to 5, comprising at least two strings of containers, means for air displacement and a single reservoir for sterile solution, the at least two strings of containers being placed in parallel, the means for air displacement being connected to the outlets of all the strings of containers via connecting means, and the single reservoir for sterile solution being optionally connected to the inlets of all the strings of containers via additional connecting means.

8. The system of one of claims 1 to 5, comprising N strings of containers (N ≥ 2), N individual means for air displacement, and a single reservoir for sterile solution, the N strings of containers being placed in parallel and each of the N individual means for air displacement being connected to the outlet of one single string of containers among the N strings of containers via connecting means, and the single reservoir for sterile solution being connected to the inlets of the N strings of containers via additional connecting means.

9. The system of one of claims 1 to 8 wherein said substantially non-deforming material is a substantially rigid material.

10. The system of one of claims 1 to 8 wherein said substantially non-deforming material is a flexible material whose deformation is limited by applied forces such as framing means or an externally applied mold which regulates the container volume.

11. The system of one of claims 1 to 10 wherein said substantially non-deforming material is sufficiently non-deforming in order for each container to be filled with a predetermined volume of said sterile solution with a better accuracy than 20%, in particular with a better accuracy than 10%, and in particular with a better accuracy than 5%.

12. The system of one of claims 1 to 11, comprising at least one reservoir for sterile solution and wherein said at least one reservoir for sterile solution is made of a flexible material.

13. The system of one of claims 2 to 12, wherein the means for air displacement comprise at least one venting bag and wherein said at least one venting bag is made of a flexible material.

14. The system of one of claims 1 to 13 wherein all containers have the same volume.

15. The system of one of claims 1 to 13 wherein at least some of the containers have different volumes.

16. The system of one of claims 1 to 15 wherein the volume of each individual container independently ranges from about 0.1 mL to about 200 mL.

17. The system of one of claims 1 to 16 wherein the number of individual containers per string of containers independently ranges from 2 to about 100.

18. The system of one of claims 1 to 17 wherein the number of strings of containers ranges from 1 to about 20.

19. The system of one of claims 1 to 18 wherein the containers are selected among the group composed of vials, formed containers, pouches, or bags.

20. The system of one of claims 1 to 19 wherein at least one of the individual containers further comprises at least one individual port per container for extracting sterile solution and optionally at least one other individual port per container for letting in air when sterile solution is extracted.

21. The system of one of claims 1 to 20 wherein the at least one string of containers is made of a single tube of constant cross-section, the containers being predefined by a specification which is indicative of the boundaries of the individual containers, each container optionally comprising one or two individual ports such as capped ports and in particular such as capped female Luer ports.

22. The system of one of claims 1 to 20 wherein the at least one string of containers is made of a single tubing comprising portions of tube alternated with flow-through couplings and in particular male-female Luer connections, the containers being partly predefined by a specification which is indicative of the boundaries of the individual containers and by said flow-through couplings, each container optionally comprising individual ports in particular for attachment to a capped sterile vent.

23. The system of one of claims 1 to 20 wherein the at least one string of containers is made of a single tubing comprising alternated portions of tubing of respectively larger and smaller cross-section, and wherein the individual containers are substantially predefined by the alternation of said portions of tubing and each individual container optionally comprises an individual port such as a capped port.

24. The system of one of claims 1 to 20 wherein the at least one string of containers is made of a substantially rigid material and the individual containers are substantially predefined as strings of formed containers by partial partition of the single tubing and optionally comprise at least one individual port such as a capped port.

25. The system of one of claims 1 to 24 wherein the containers are designed with materials and shapes which are resistant to cracking when cycled through freezing and thawing and which does not entrap air pockets within the containers their access ports or the fill ports.

26. A method for preparing individual containers to be filled with a substantially accurately predetermined volume of a sterile solution comprising the steps of :
- providing a system according to any one of claims 1 to 25 comprising at least one reservoir for sterile solution and additional connecting means, said at least one reservoir for sterile solution being connected to the single inlet of each of the at least one string of containers via said additional connecting means ;
- injecting the sterile solution from said at least one reservoir filled with sterile solution into the at least one string of containers via the single inlet of the at least one string of containers, so as to provide individual containers filled with a predetermined volume of sterile solution;
- sealing the at least one string of containers by sealing means at a plurality of positions, so as to provide filled, separate, individual containers ; and
- optionally detaching each individual container from the at least one string of containers by detaching means, so as to provide filled, separate, detached, individual containers.

27. The method of claim 26, wherein the system remains closed relative to the ambient environment during the steps of injecting the sterile solution into the at least one string of containers and of sealing the at least one string of containers, so that there is no contact between the sterile solution and air, when present, inside the system on the one hand and the ambient environment outside the system on the other hand.

28. The method of claim 26, wherein the system remains open relative to the ambient environment until the sealing of the at least one string of containers, the sterility of the sterile solution being maintained at all steps via at least one filtering means.

29. The method of one of claims 26 to 28, wherein the system comprises at least one venting bag for displaced air which is connected to the outlets of the at least one string of containers and which is mostly empty prior to injecting the sterile solution into the at least one string of containers.

30. The method of one of claims 26 to 29, wherein the injection of the sterile solution into the at least one string of containers is made from the top to the bottom or from the bottom to the top by means of hydrostatic pressure or of filling means such as a peristaltic pump.

31. The method of one of claims 26 to 30, wherein the injection of the sterile solution is made from the bottom to the top, whereby air bubbles in the containers are substantially avoided.

32. The method of one of claims 26 to 31, wherein the sealing of the containers of each of the at least one string of containers is carried out by sealing means either after the filling of said string of containers, or after the complete filling of all of the at least one string of containers.

33. The method of one of claims 26 to 32, which comprises the additional final step of freezing and optionally thawing the sterile solution inside the containers, optionally more than once, without stress fractures appearing on said containers.

34. The method of one of claims 26 to 33, wherein the individual containers filled with a predetermined volume of sterile solution obtained after the step of injecting the sterile solution or the filled, separate, individual containers obtained after the step of sealing or the filled, separate, detached, individual containers obtained after the optional step of detaching are subsequently used for cellular or clinical applications.
